(19) Europäisches Patentamt
European Patent Office
Office européen des brevets

(11) **EP 4 029 495 A1**

(12) **EUROPEAN PATENT APPLICATION**
published in accordance with Art. 153(4) EPC

(43) Date of publication:
**20.07.2022 Bulletin 2022/29**

(21) Application number: **20863752.0**

(22) Date of filing: **09.09.2020**

(51) International Patent Classification (IPC):
*A61K 9/50* (2006.01)    *A61K 47/14* (2017.01)
*A61K 31/451* (2006.01)

(52) Cooperative Patent Classification (CPC):
**A61K 9/50; A61K 31/451; A61K 47/14**

(86) International application number:
**PCT/JP2020/034123**

(87) International publication number:
**WO 2021/049529 (18.03.2021 Gazette 2021/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.09.2019  JP 2019166802**

(71) Applicant: **SEKISUI CHEMICAL CO., LTD.**
**Osaka-shi**
**Osaka**
**530-8565 (JP)**

(72) Inventors:
• **NAKAMURA, Yuuta**
**Mishima-gun, Osaka 618-0021 (JP)**

• **MATSUMOTO, Izumi**
**Mishima-gun, Osaka 618-0021 (JP)**
• **AKAMINE, Takayuki**
**Mishima-gun, Osaka 618-0021 (JP)**
• **KAWAMURA, Daichi**
**Mishima-gun, Osaka 618-0021 (JP)**
• **TONE, Saori**
**Mishima-gun, Osaka 618-0021 (JP)**
• **LI, Yan**
**Mishima-gun, Osaka 618-0021 (JP)**
• **OKAMOTO, Naoki**
**Mishima-gun, Osaka 618-0021 (JP)**

(74) Representative: **Ter Meer Steinmeister & Partner**
**Patentanwälte mbB**
**Nymphenburger Straße 4**
**80335 München (DE)**

(54) **FORMULATION**

(57)    Provided is a formulation that can improve all of transdermal absorbability of an active ingredient, immediate effect in transdermal absorption, and reduction of skin irritation, to high levels. A formulation containing: a base; and a core-shell structure having a core portion containing an active ingredient and a shell portion containing a surfactant; in which the core portion is a solid, the surfactant contains a compound having two or more fatty acid bonds and a compound having one fatty acid bond, and a proportion of the compound having two or more fatty acid bonds in the surfactant is 5% by weight or more and 75% by weight or less.

[FIG. 1.]

EP 4 029 495 A1

**Description**

**TECHNICAL FIELD**

The present invention relates to a formulation containing an active ingredient and a surfactant.

**BACKGROUND ART**

In the fields of external medicines, cosmetics, and the like, a technique for transdermally absorbing an active ingredient such as a drug has been developed. The transdermal absorption process of an active ingredient may be affected by skin barrier function, metabolism, and the like, and it is known that these effects vary depending on the drug.
Patent Document 1 below discloses a core-shell structure including a core portion containing an active ingredient and a shell portion containing a surfactant having an HLB value of 4 to 14. The surfactant is considered to have a saturated hydrocarbon group having 7 to 15 carbon atoms or an unsaturated hydrocarbon group having 7 to 17 carbon atoms.

**Related Art Document**

**Patent Document**

Patent Document 1: WO 2018/147333 A

**SUMMARY OF THE INVENTION**

**PROBLEMS TO BE SOLVED BY THE INVENTION**

In Patent Document 1, a formulation containing a core-shell structure as described above is considered to be excellent in immediate effect in transdermal absorption of an active ingredient. However, when an attempt is made to enhance the transdermal absorbability and immediate effect of the active ingredient by the formulation of Patent Document 1, skin irritation may be enhanced. Therefore, it is required to improve all of transdermal absorbability of an active ingredient, immediate effect in transdermal absorption, and reduction of skin irritation, to higher levels.
An object of the present invention is to provide a formulation that can improve all of transdermal absorbability of an active ingredient, immediate effect in transdermal absorption, and reduction of skin irritation, to high levels.

**MEANS FOR SOLVING THE PROBLEMS**

A formulation according to the present invention contains a base, and a core-shell structure having a core portion containing an active ingredient and a shell portion containing a surfactant, in which the core portion is a solid, the surfactant contains a compound having two or more fatty acid bonds and a compound having one fatty acid bond, and a proportion of the compound having two or more fatty acid bonds in the surfactant is 5% by weight or more and 75% by weight or less.
In a specific aspect of the formulation according to the present invention, the proportion of the compound having two or more fatty acid bonds in the surfactant is 10% by weight or more and 70% by weight or less.
In another specific aspect of the formulation according to the present invention, the compound having two or more fatty acid bonds contains at least one of an ester of two or more fatty acids and a polyhydric alcohol and an ester of two or more fatty acids and a sugar or sugar derivative.
In still another specific aspect of the formulation according to the present invention, the compound having two or more fatty acid bonds contains at least one of an ester of two or more fatty acids and glycerin and an ester of two or more fatty acids and sorbitan. Among them, it is more preferred that the compound having two or more fatty acid bonds contains an ester of two or more fatty acids and glycerin.
In still another specific aspect of the formulation according to the present invention, the surfactant has a weighted average value of an HLB value of 4 or more and 14 or less.
In still another specific aspect of the formulation according to the present invention, the surfactant has a saturated hydrocarbon group having 7 to 15 carbon atoms or an unsaturated hydrocarbon group having 7 to 17 carbon atoms.
In still another specific aspect of the formulation according to the present invention, the mass ratio between the active ingredient and the surfactant (active ingredient : surfactant) is 1 : 0.1 to 1 : 10.
In still another specific aspect of the formulation according to the present invention, the base is an oily base.

**EFFECT OF THE INVENTION**

According to the present invention, it is possible to provide a formulation that can improve all of transdermal absorbability of an active ingredient, immediate effect in transdermal absorption, and reduction of skin irritation, to high levels.

**BRIEF DESCRIPTION OF DRAWINGS**

[Fig. 1] Fig. 1 is a schematic cross-sectional view for illustrating a core-shell structure according to an embodiment of the present invention.
[Fig. 2] Fig. 2 is a schematic cross-sectional view of a drug skin permeation test cell.
[Fig. 3] Fig. 3 is a schematic view for explaining a measurement method of lag time.

**MODES FOR CARRYING OUT THE INVENTION**

Hereinafter, the details of the present invention will be described.

The formulation of the present invention contains a base and a core-shell structure. The core-shell structure has a core portion containing an active ingredient and a shell portion containing a surfactant. The core portion is a solid. The surfactant contains a compound having two or more fatty acid bonds and a compound having one fatty acid bond. Also, in the present invention, a proportion of the compound having two or more fatty acid bonds in the surfactant is 5% by weight or more and 75% by weight or less.

Since the formulation of the present invention has the above-described configuration, it is possible to improve all of transdermal absorbability of an active ingredient, immediate effect in transdermal absorption, and reduction of skin irritation, to high levels.

Conventionally, when an attempt is made to enhance the transdermal absorbability and immediate effect of the active ingredient, skin irritation may be enhanced.

The present inventors have focused on the number of fatty acid bonds in a surfactant, and have found that the surfactant contains a compound having two or more fatty acid bonds and a compound having one fatty acid bond, and in particular, the proportion of the compound having two or more fatty acid bonds in the surfactant is set to 5% by weight or more and 75% by weight or less, whereby it is possible to improve all of transdermal absorbability of an active ingredient, immediate effect in transdermal absorption, and reduction of skin irritation, to high levels.

In the present specification, the proportion of the compound having two or more fatty acid bonds in the surfactant can be measured, for example, by gel permeation chromatography (GPC). Specifically, using an elution curve of a surfactant having a known ratio of the number of fatty acid bonds of less than 2 and the number of fatty acid bonds of 2 or more obtained by GPC as a standard curve, peaks of a compound having less than two fatty acid bonds and the compound having two or more fatty acid bonds, of which ratio is unknown, are identified, and the proportion can be determined from the peak intensity. For example, when the surfactant is composed of monoglyceride and diglyceride, peaks of the monoglyceride and the diglyceride are identified, and a proportion of the diglyceride (proportion of the compound having two or more fatty acid bonds) can be determined from the peak intensity. Similarly, a proportion of the monoglyceride (proportion of the compound having one fatty acid bond) can be determined from the peak intensity.

Hereinafter, each component constituting the formulation of the present invention will be described in more detail.

(Active ingredient)

The formulation of the present invention contains an active ingredient. Specific examples of the active ingredient include, but are not particularly limited to, dementia therapeutic agents, antiepileptic agents, antidepressants, antiparkinsonian agents, anti-allergic agents, anticancer agents, antidiabetics, antihypertensive agents, respiratory disease drugs, erectile dysfunction drugs, skin disease therapeutic agents, local analgesics, and the like. The active ingredients may be used alone, or a plurality of types may be used in combination.

More specific examples include memantine, donepezil, diphenhydramine, vardenafil, octreotide, rivastigmine, galantamine, nitroglycerin, lidocaine, fentanyl, male hormones, female hormones, nicotine, clomipramine, nalfurafine, metoprolol, fesoterodine, tandospirone, beraprost sodium, taltirelin, lurasidone, nefazodone, rifaximin, benidipine, doxazosin, nicardipine, formoterol, lomerizine, amlodipine, teriparatide, bucladesine, cromoglicic acid, lixisenatide, exenatide, liraglutide, lanreotide, glucagon, oxytocin, calcitonin, elcatonin, glatiramer, risedronic acid, diclofenac, ascorbic acid, pharmaceutically acceptable salts thereof, and the like.

The pharmaceutically acceptable salt is not particularly limited, and any of acid salts and basic salts can be employed. Examples of the acid salts include inorganic acid salts such as hydrochloride, hydrobromide, sulfate, nitrate, and phosphate, and organic acid salts such as acetate, propionate, tartrate, fumarate, maleate, malate, citrate, methanesulfonate, benzenesulfonate, and p-toluenesulfonate. In addition, examples of the basic salts include alkali metal salts such as sodium salts and potassium salts, alkaline-earth metal salts such as calcium salts and magnesium salts, and the like. Specific examples of active ingredient salts include memantine hydrochloride, donepezil hydrochloride, rivastigmine tartrate, galantamine hydrobromide, clomipramine hydrochloride, diphenhydramine hydrochloride, nalfurafine hydrochloride, metoprolol tartrate, fesoterodine fumarate, vardenafil hydrochloride hydrate, nalfurafine hydrochloride, tandospirone citrate, beraprost sodium, lurasidone hydrochloride, nefazodone hydrochloride, benidipine hydrochloride, doxazosin mesylate, nicardipine hydrochloride, formoterol fumarate, lomerizine hydrochloride, amlodipine besylate, and the like.

An active ingredient to be formulated into a cosmetic is not particularly limited as long as skin permeation is required. Examples thereof include vitamin ingredients such as vitamin C and vitamin E, moisturizing ingredients such as hyaluronic acid, ceramide, and collagen, skin-whitening ingredients such as tranexamic acid and arbutin, hair growth ingredients such as minoxidil, beauty ingredients such as FGF (fibroblast growth factor) and EGF (epidermal growth factor), salts and derivatives thereof, and the like.

The active ingredient is preferably hydrophilic. When the active ingredient is a hydrophilic drug, a drug required to have a systemic effect or local effect is usually used.

The active ingredient, if easily transdermally absorbed, is preferable. The active ingredient is preferably, but is not particularly limited to, a compound exhibiting an octanol/water partition coefficient of -2 to 6. In this case, skin permeability of the active ingredient is further enhanced. From the viewpoint of further enhancing the skin permeability of the active ingredient, the octanol/water partition coefficient is preferably -1 or more, and more preferably 0 or more. Also, the octanol/water partition coefficient of the active ingredient is preferably 4 or less, and more preferably 1 or less. When the octanol/water partition coefficient of the active ingredient is equal to or less than the above upper limit, the skin permeability of the active ingredient is further enhanced.

Note that, in the present invention, the octanol/water partition coefficient is determined based on active ingredient concentration of each phase by adding an active ingredient to a flask containing octanol and an aqueous buffer of pH 7, then shaking the flask, and determining the concentration. Specifically, it can be determined by calculation using an equation: Octanol/water partition coefficient = $Log_{10}$ (concentration in octanol phase/concentration in aqueous phase).

In the present invention, the molecular weight of the active ingredient is not particularly limited. The molecular weight of the active ingredient is preferably 250 g/mol or more, more preferably 300 g/mol or more, preferably 7,500 g/mol or less, more preferably 6,500 g/mol or less, and further preferably 1,500 g/mol or less.

The content of the active ingredient in the formulation of the present invention, although depending on the type of the active ingredient, is, for example, preferably 1% by weight or more and 40% by weight or less, and more preferably 5% by weight or more and 30% by weight or less as a raw material weight. The raw material weight is a value based on the total weight of all raw materials contained in the formulation.

Note that, the formulation of the present invention may contain two or more active ingredients as the active ingredients as necessary.

(Surfactant)

In the present invention, the surfactant contains a compound having two or more fatty acid bonds. The compound having two or more fatty acid bonds is a compound having a structure derived from two or more fatty acids in the molecule.

Examples of the compound having two or more fatty acid bonds include esters of two or more fatty acids and a polyhydric alcohol, esters of two or more fatty acids and a sugar or sugar derivative, and the like.

Examples of the polyhydric alcohol include glycerin, polyglycerin, polyoxyethylene glycerin, and the like. The ester of two or more fatty acids and a polyhydric alcohol is not particularly limited, but an ester of two or more fatty acids and glycerin is preferable.

Examples of the sugar or sugar derivative include sucrose, sorbitan, polyoxyethylene sorbitan, and the like. The ester of two or more fatty acids and a sugar is not particularly limited, but an ester of two or more fatty acids and sucrose or an ester of two or more fatty acids and sorbitan is preferable.

Among the compounds having two or more fatty acid bonds described above, an ester of two or more fatty acids and glycerin or an ester of two or more fatty acids and sorbitan is more preferable. In this case, it is possible to improve all of transdermal absorbability of an active ingredient, immediate effect in transdermal absorption, and reduction of skin irritation, to even higher levels.

The compound having two or more fatty acid bonds is not particularly limited, but is preferably a compound having two or more and four or less fatty acid bonds, more preferably a compound having two or more and three or less fatty acid bonds, and further preferably a compound having two fatty acid bonds. In this case, it is possible to improve all of transdermal absorbability of an active ingredient, immediate effect in transdermal absorption, and reduction of skin irritation, to even higher levels.

As the compound having two or more fatty acid bonds, an ester of two fatty acids and glycerin (diglyceride) or an ester of two fatty acids and sorbitan is particularly preferable, and an ester of two fatty acids and glycerin (diglyceride) is especially preferable. In this case, it is possible to improve all of transdermal absorbability of an active ingredient, immediate effect in transdermal absorption, and reduction of skin irritation, to even higher levels.

Note that, the compounds having two or more fatty acid bonds may be used singly, or a plurality of types may be used in combination.

Examples of the fatty acid constituting the compound having two or more fatty acid bonds include caproic acid, caprylic acid, capric acid, lauric acid, myristic acid, palmitic acid, stearic acid, behenic acid, undecylenic acid, ricinoleic acid, oleic acid, linoleic acid, linolenic acid, ricinolenic acid, erucic acid, beef tallow, lard, coconut oil, palm oil, palm kernel oil, olive oil, rapeseed oil, rice bran oil, soybean oil, castor oil, and the like. These may be used singly, or a plurality of types may be used in combination.

Specific examples of the compound having two or more fatty acid bonds include diglyceride caprylate, diglyceride caprate, diglyceride stearate, diglyceryl triisostearate, polyglyceryl distearate, polyglyceryl diisostearate, decaglyceryl triisostearate, polyglyceryl trioleate, decaglyceryl pentastearate, polyglyceryl pentaoleate, polyoxyethylene glyceryl triisostearate, sorbitan distearate, sorbitan sesquistearate, sorbitan tristearate, sorbitan sesquioleate, sorbitan trioleate, polyoxyethylene sorbitan tristearate, and polyoxyethylene sorbitan trioleate.

In the present invention, a proportion of the compound having two or more fatty acid bonds in the surfactant is 5% by weight or more, preferably 10% by weight or more, more preferably 20% by weight or more, further preferably 25% by weight or more, and particularly preferably 30% by weight or more, and is 75% by weight or less, preferably 70% by weight or less, more preferably 65% by weight or less, and further preferably 60% by weight or less. When the proportion of the compound having two or more fatty acid bonds in the surfactant is equal to or more than the above lower limit, the immediate effect can be further enhanced in transdermal absorption. Also, when the proportion of the compound having two or more fatty acid bonds in the surfactant is equal to or less than the above upper limit, the skin irritation can be further reduced.

As a surfactant containing the compound having two or more fatty acid bonds in the above proportion, for example, a commercially available surfactant can be used. In addition, a surfactant of which proportion of the compound having two or more fatty acid bonds has been adjusted within the above range using a plurality of commercially available products having different proportions of the compound having two or more fatty acid bonds may be used.

Therefore, examples of the component other than the compound having two or more fatty acid bonds contained in the surfactant include compounds having one fatty acid bond such as monoglyceride, contained in commercially available surfactants.

In the present invention, the proportion of the compound having one fatty acid bond in the surfactant is preferably 10% by weight or more, more preferably 20% by weight or more, further preferably 30% by weight or more, preferably 95% by weight or less, more preferably 80% by weight or less, and further preferably 70% by weight or less. When the proportion of the compound having one fatty acid bond in the surfactant is equal to or more than the above lower limit, the transdermal absorbability and immediate effect of the active ingredient are further easily enhanced. In addition, when the proportion of the compound having one fatty acid bond in the surfactant is equal to or less than the above upper limit, the skin irritation is more easily reduced.

However, the formulation of the present invention may further contain other surfactants. The other surfactant may be any of a nonionic surfactant, an anionic surfactant, a cationic surfactant, or an amphoteric surfactant.

In the present invention, an HLB value of the surfactant is preferably 4 or more and 14 or less.

An HLB (abbreviation of Hydrophile Lypophile Balance) value in the present invention, which is an index showing that an emulsifier is hydrophilic or lipophilic, takes a value of 0 to 20. A smaller HLB value indicates a higher lipophilicity.

In the present invention, the HLB value is calculated by the following Griffin equation.

$$\text{HLB value} = 20 \times \{(\text{Molecular weight of hydrophilic moiety})/(\text{Total molecular weight})\}$$

A weighted average value of the HLB value can be calculated, for example, using the following calculation equation.

A calculation equation for a weighted average value, when surfactants having HLB values of A, B, and C are used in weights of x, y, and z, respectively, is as follows:

$$(xA + yB + zC)/(x + y + z).$$

The HLB value of the surfactant or, when a plurality of surfactants is contained, the weighted average value of the HLB value is 4 or more and 14 or less, and more preferably 5 or more and 12 or less. In this case, the immediate effect can be further improved in transdermal absorption.

The surfactant may have at least one of a saturated hydrocarbon group such as an alkyl group and an unsaturated hydrocarbon group such as an alkenyl group or an alkynyl group.

The number of carbon atoms in the saturated hydrocarbon group is preferably 7 or more and 15 or less, and more preferably 7 or more and 11 or less. In this case, the immediate effect can be further improved in transdermal absorption.

The number of carbon atoms in the unsaturated hydrocarbon group is preferably 7 or more and 17 or less, more preferably 7 or more and 13 or less, and further preferably 7 or more and 11 or less. In this case, the immediate effect can be further improved in transdermal absorption.

When a surfactant contains a plurality of hydrocarbon groups, the hydrocarbon group of which content in the surfactant is the highest is taken as the hydrocarbon group of the surfactant.

Particularly when a surfactant contains a plurality of hydrocarbon groups having different numbers of carbon atoms, the number of carbon atoms in the hydrocarbon group of which content in the surfactant is the highest is taken as the number of carbon atoms in the hydrocarbon group of the surfactant.

Further, when a plurality of surfactants is contained, the number of carbon atoms in the hydrocarbon group of which content in the plurality of surfactants is the highest is taken as the number of carbon atoms in the hydrocarbon group in the surfactant of the present invention.

In the present invention, the content of the surfactant can be appropriately set within a range in which the effect of the present invention is exerted, but is preferably 2% by weight to 40% by weight, and more preferably 5% by weight to 30% by weight as a raw material weight. The raw material weight is a value based on the total weight of all raw materials contained in the formulation.

In the present invention, the lower limit of the mass ratio between the active ingredient and the surfactant (active ingredient : surfactant) is preferably 1 : 0.1 or more, more preferably 1 : 0.3 or more, and further preferably 1 : 0.5 or more. The upper limit of the mass ratio between the active ingredient and the surfactant (active ingredient : surfactant) is preferably 1 : 10 or less, more preferably 1 : 5 or less, and further preferably 1 : 2 or less. In particular, in the present invention, the mass ratio between the active ingredient and the surfactant (active ingredient : surfactant) is preferably 1 : 0.1 to 1 : 10, more preferably 1 : 0.5 to 1 : 10, further preferably 1 : 0.5 to 1 : 5, and particularly preferably 1 : 0.5 to 1 : 2. In this case, the immediate effect can be further enhanced in transdermal absorption.

(Core-shell structure)

The formulation of the present invention contains a core-shell structure having a core portion containing an active ingredient and a shell portion containing a surfactant. Therefore, it is possible to improve all of transdermal absorbability of an active ingredient, immediate effect in transdermal absorption, and reduction of skin irritation, to even higher levels.

In the present invention, the core portion and the shell portion may be bound by intermolecular forces or the like to form an assembly. However, from the viewpoint of further enhancing the transdermal absorbability and immediate effect of the active ingredient, at least a part of the surface of the core portion is preferably covered with the shell portion.

More specifically, 30% or more of the surface of the core portion is preferably covered with the shell portion. More preferably 50% or more, further preferably 70% or more, further preferably 85% or more, particularly preferably 95% or more, and most preferably 99% or more of the surface of the core portion is covered with the shell portion. However, the surface of the core portion may be completely covered with the shell portion. Since the core-shell structure has the above-described configuration, for example, when the core-shell structure is applied to skin, the transdermal absorbability and immediate effect of the active ingredient contained in the core portion can be further enhanced.

The core portion is a solid. Since the core portion is solid, stability in a base phase described later can be further improved. In this case, a formulation having a S/O (Solid in Oil) type structure can be formed by dispersing the core-shell structure in a base phase which is an oil phase.

As described in the section of a production method described later, since the core-shell structure is obtained by drying W/O emulsion and removing solvents (aqueous solvent and oily solvent), the core portion is a solid (S of S/O (Solid in Oil) type). It is preferable that moisture is substantially completely removed by a step of drying the W/O emulsion. Specifically, for example, the water content is preferably 5% by weight or less, more preferably 2% by weight or less, further preferably 1% by weight or less, and particularly preferably 0.5% by weight or less as measured by Karl Fischer method. Thus, the core-shell structure is different from the W/O emulsion.

Hereinafter, an example of the core-shell structure will be described with reference to a drawing.

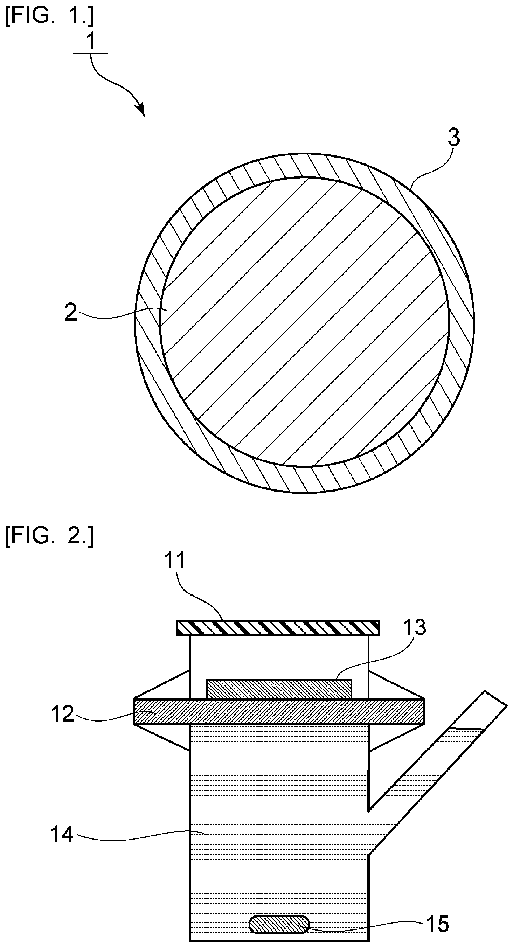

Fig. 1 is a schematic cross-sectional view for illustrating a core-shell structure according to an embodiment of the present invention.

As shown in Fig. 1, a core-shell structure 1 includes a core portion 2 and a shell portion 3. The surface of the core portion 2 is covered with the shell portion 3.

However, the shape of the core-shell structure is not limited to such spherical particle. The core-shell structure may be, for example, a particle having a rod shape, a cubic shape, a lens shape, a micelle shape, a lamellar shape, a hexagonal shape, a bicelle shape, a sponge shape, or a sea urchin-like shape, or may be an indefinite shape. As described above, the shape of the core-shell structure is not particularly limited. However, as described above, at least a part of the surface of the core portion is preferably covered with the shell portion.

In addition, the size of the core-shell structure is not particularly limited. From the viewpoint of further enhancing the transdermal absorbability and immediate effect of the active ingredient, the average size of the core-shell structure can be set to preferably 1 nm to 100 $\mu$m.

Note that, in the present invention, the average size of the core-shell structure is a number average diameter calculated by a dynamic light scattering method during dispersion of a solvent (for example, squalane or the like).

The content ratio of the core-shell structure in the formulation of the present invention is not particularly limited, but can be set to preferably 10% by mass or more and 70% by mass or less, and more preferably 20% by mass or more and 50% by mass or less.

In the present invention, the lower limit of the mass ratio between the core portion and the shell portion (core portion : shell portion) in the core-shell structure is preferably 1 : 0.1 or more, more preferably 1 : 0.3 or more, and further preferably 1 : 0.5 or more. The upper limit of the mass ratio between the core portion and the shell portion (core portion : shell portion) is preferably 1 : 10 or less, more preferably 1 : 5 or less, and further preferably 1 : 2 or less. Also, in the present invention, the mass ratio between the core portion and the shell portion (core portion : shell portion) in the core-shell structure is not particularly limited, and is preferably 1 : 0.1 to 1 : 10, more preferably 1 : 0.5 to 1 : 10, further preferably 1 : 0.5 to 1 : 5, and particularly preferably 1 : 0.5 to 1 : 2. In this case, the transdermal absorbability and immediate effect of the active ingredient can be further enhanced.

Other additive components;

The core-shell structure may further contain at least one other component, in addition to the active ingredient and the surfactant. Examples of the other component include, but are not particularly limited to, a stabilizing agent, a transdermal absorption enhancer, a skin irritation reducing agent, an antiseptic, an analgesic, and the like.

The stabilizing agent has an action of stabilizing a particle structure. Examples of the stabilizing agent include, but are not particularly limited to, polysaccharides, proteins, hydrophilic polymer materials, and the like. One or two or more stabilizing agents may be contained. The content of the stabilizing agent can be set appropriately depending on the type thereof. The stabilizing agent can be formulated so that, for example, the weight ratio between the active ingredient and the stabilizing agent (active ingredient : stabilizing agent) is 1 : 0.1 to 1 : 10.

Examples of the transdermal absorption enhancer include, but are not particularly limited to, higher alcohols, N-acyl sarcosine and salts thereof, higher monocarboxylic acids, higher monocarboxylic acid esters, aromatic monoterpene fatty acid esters, dicarboxylic acids having 2 to 10 carbon atoms and salts thereof, polyoxyethylene alkyl ether phosphoric acid esters and salts thereof, lactic acid, lactic acid esters, citric acid, and the like. One or two or more transdermal absorption enhancers may be contained. The content of the transdermal absorption enhancer can be set appropriately depending on the type thereof. The transdermal absorption enhancer can be formulated so that, for example, the weight ratio between the active ingredient and the transdermal absorption enhancer (active ingredient : transdermal absorption enhancer) is 1 : 0.01 to 1 : 50.

Examples of the skin irritation reducing agent include, but are not particularly limited to, hydroquinone glycosides, pantethine, tranexamic acid, lecithin, titanium oxide, aluminum hydroxide, sodium nitrite, sodium hydrogen nitrite, soybean lecithin, methionine, glycyrrhetinic acid, BHT, BHA, vitamin E and derivatives thereof, vitamin C and derivatives thereof, benzotriazole, propyl gallate, mercaptobenzimidazole, and the like. One or two or more skin irritation reducing agents may be contained. The content ratio of the skin irritation reducing agent can be set appropriately depending on the type thereof. The skin irritation reducing agent can be formulated such that its content is, for example, 0.1% by weight to 50% by weight relative to the entire core-shell structure.

Examples of the antiseptic include, but are not particularly limited to, methyl paraoxybenzoate, propyl paraoxybenzoate, phenoxy ethanol, thymol, and the like. The content ratio of the antiseptic in the core portion can be set appropriately depending on the type thereof. The antiseptic can be formulated such that its content is, for example, 0.01% by weight to 10% by weight relative to the entire core-shell structure. One or two or more antiseptics may be contained.

Examples of the analgesic include, but are not particularly limited to, local anesthetics such as procaine, tetracaine, lidocaine, dibucaine and prilocaine, salts thereof, and the like. One or two or more analgesics may be contained. The content ratio of the analgesic in the core-shell structure can be set appropriately depending on the type thereof. The analgesic can be formulated such that its content is, for example, 0.1% by weight to 30% by weight relative to the entire core-shell structure.

Production method;

The core-shell structure can be produced by, but not particularly limited to, for example, a method including a step of drying a W/O emulsion containing an active ingredient in an aqueous phase.

The W/O emulsion is not particularly limited, as long as it is a so-called water-in-oil emulsion, specifically, an emulsion in which droplets of an aqueous solvent are dispersed in an oily solvent.

The W/O emulsion containing an active ingredient in an aqueous phase can be obtained, for example, by mixing an aqueous solvent such as water or a buffer aqueous solution containing an active ingredient with an oily solvent such as cyclohexane, hexane or toluene containing a surfactant. The aqueous solvent containing an active ingredient may contain an additive component such as a stabilizing agent, an absorption enhancer or an irritation reducing agent as necessary, in addition to the active ingredient. Also, the oily solvent containing a surfactant may contain an additive component such as an irritation reducing agent, an analgesic, an absorption enhancer or a stabilizing agent as necessary, in addition to the surfactant. The mixing method is not particularly limited, as long as it is a method that can form a W/O emulsion, and examples thereof include stirring with a homogenizer or the like.

Conditions for stirring with a homogenizer are, for example, about 5,000 rpm to 50,000 rpm, and preferably about 10,000 rpm to 30,000 rpm.

The lower limit of the mass ratio between the active ingredient and the surfactant (active ingredient : surfactant) in the W/O emulsion is preferably 1 : 0.1 or more, more preferably 1 : 0.3 or more, and further preferably 1 : 0.5 or more. The upper limit of the mass ratio between the active ingredient and the surfactant (active ingredient : surfactant) in the W/O emulsion is preferably 1 : 10 or less, more preferably 1 : 5 or less, and further preferably 1 : 2 or less. The mass ratio between the active ingredient and the surfactant (active ingredient : surfactant) in the W/O emulsion is preferably 1 : 0.1 to 1 : 10, more preferably 1 : 0.5 to 1 : 10, further preferably 1 : 0.5 to 1 : 5, and particularly preferably 1 : 0.5 to 1 : 2.

A method for drying the W/O emulsion containing an active ingredient in an aqueous phase is not particularly limited, as long as it is a method that can remove the solvents (aqueous solvent and oily solvent) contained in the emulsion. Examples of the method for drying the W/O emulsion include freeze drying, reduced pressure drying and the like, and preferably freeze drying.

Also, the method described above preferably further includes a step of heat-treating the W/O emulsion or a dried substance of the W/O emulsion, from the viewpoint of further reducing the number average particle diameter of the core-shell structure to be obtained. Heat treatment temperature is, for example, 30°C to 60°C, preferably 35°C to 50°C, and more preferably 35°C to 45°C.

Heat treatment time is adjusted appropriately in accordance with the heat treatment temperature, and is, for example, 1 day to 30 days, preferably 2 days to 15 days, and more preferably 3 to 7 days.

In addition, as another method for further reducing the number average particle diameter of the obtained core-shell structure, methods of dispersing the W/O emulsion or a dried substance of the W/O emulsion in a solvent or the like as necessary, and then filtering the dispersion with a filter or the like, or a subjecting the dispersion to centrifugation separation. In the case of filtration through a filter, a pore diameter of the filter is, for example, 1 $\mu$m or less, preferably 0.2 $\mu$m or less, and more preferably 0.1 $\mu$m or less.

(Formulation)

The formulation of the present invention can be used in wide variety of applications intended for transdermal adsorption or transmucosal absorption, for example, external medicines such as external skin medicines, eye drops, nasal sprays, suppositories, and oral cavity drugs, cosmetics, and injections, depending on the type of the active ingredient.

The formulation of the present invention is usually sustained, but not particularly limited to, for 1 day to 1 week, and is used by once-a-day to once-a-week administration in a preferred embodiment.

When the formulation of the present invention is an external medicine, a target disease differs depending on the type of the active ingredient.

The formulation of the present invention is not particularly limited, and can be used as an adhesive preparation such as a tape preparation such as a plaster preparation or a tape preparation such as a plaster (reservoir type, matrix type, etc.), a poultice, a patch or a microneedle, an ointment, an external liquid preparation such as a liniment or a lotion, a spray preparation such as an external aerosol or a pump spray preparation, a cream, a gel, an eye drop, an eye ointment, a nasal drop, a suppository, a rectal semisolid, an enema agent, an oral agent, an injection, or the like.

The formulation of the present invention preferably has a water content of 20% by mass or less, and more preferably contains substantially no water. This makes it possible to further enhance shape retainability when the core-shell structure is used. In addition, in combination with the intrinsic shape retainability of the core-shell structure, leakage of the active ingredient from the core-shell structure, eventually, crystallization of the active ingredient can be further suppressed, and as a result, it is possible to exert further enhanced transdermal absorbability. From this viewpoint, the formulation of the present invention is preferably used as an agent of which water content is adjusted to 20% by mass or less. It is more preferable that the formulation of the present invention is more preferably used as an agent containing substantially no water. The formulation of the present invention is preferably used as, for example, a plaster preparation, a patch, an ointment, a gel, or the like.

(Base phase)

The formulation of the present invention may contain a base phase, and the base phase may contain a core-shell structure. At this time, the core-shell structure is preferably dispersed or dissolved in the base phase.

The base is not particularly limited, and can be widely selected from those that can be used as, in particular, pharmaceuticals such as external medicines and cosmetics.

As described above, in the core-shell structure, the core portion is solid. Therefore, when the base phase is an oil phase, an S/O (Solid in Oil) type formulation can be formed by dispersing the core-shell structure in the base phase which is an oil phase. The S/O type formulation can be obtained, for example, by dispersing particles obtained by the above-described production method in the oil phase.

The base can be appropriately selected from those suitable for dispersing or dissolving the core-shell structure depending on the intended use and the like, and is not particularly limited.

In addition, a plurality of types of bases may be used in combination.

The base is not particularly limited, and examples thereof include oily bases, aqueous bases, and the like. Among them, the base is preferably an oily base. When the base is an oily base, a formulation having an S/O (Solid in Oil) type structure can be formed by dispersing the core-shell structure in the oily base. The formulation having an S/O (Solid in Oil) type structure can be produced, for example, by a method including a step of drying a W/O emulsion containing an active ingredient in an aqueous phase as described above.

Examples of the oily base include vegetable oils, animal oils, neutral lipids, synthetic fats and oils, sterol derivatives, waxes, hydrocarbons, monoalcohol carboxylic acid esters, oxyacid esters, polyhydric alcohol fatty acid esters, silicones, higher alcohols, higher fatty acids, fluorine-based oils, and the like. Examples of the aqueous base include water, (polyhydric) alcohols, and the like.

Examples of the vegetable oil include, but are not particularly limited to, soybean oil, sesame oil, olive oil, coconut oil, palm oil, rice oil, cottonseed oil, sunflower oil, rice bran oil, cacao butter, corn oil, safflower oil, castor oil, rapeseed oil, and the like.

Examples of the animal oil include, but are not particularly limited to, mink oil, turtle oil, fish oil, cow oil, horse oil, pig oil, shark squalane, and the like.

Examples of the neutral lipid include, but are not particularly limited to, triolein, trilinolein, trimyristin, tristearin, triarachidonin, and the like.

Examples of the synthetic oil and fat include, but are not particularly limited to, phospholipids, azone, and the like.

Examples of the sterol derivative include, but are not particularly limited to, dihydrocholesterol, lanosterol, dihydrolanosterol, phytosterol, cholic acid, cholesteryl linoleate, and the like.

Examples of the waxes include candelilla wax, carnauba wax, rice wax, Japan wax, beeswax, montan wax, ozokerite, ceresin, paraffin wax, microcrystalline wax, petrolatum, Fischer-Tropsch wax, polyethylene wax, ethylene-propylene copolymers, and the like.

Examples of the hydrocarbons include liquid paraffin (mineral oil), heavy liquid isoparaffin, light liquid isoparaffin, $\alpha$-olefin oligomer, polyisobutene, hydrogenated polyisobutene, polybutene, squalane, olive-derived squalane, squalene, vaseline, solid paraffin, and the like.

Examples of the monoalcohol carboxylic acid esters include octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, lanolin fatty acid octyldodecyl, hexyldecyl dimethyloctanoate, octyldodecyl erucate, hydrogenated castor oil isostearate, ethyl oleate, avocado oil fatty acid ethyl, isopropyl myristate, isopropyl palmitate, octyl palmitate, isopropyl isostearate, lanolin fatty acid isopropyl, diethyl sebacate, diisopropyl sebacate, dioctyl sebacate, diisopropyl adipate, dibutyloctyl sebacate, diisobutyl adipate, dioctyl succinate, triethyl citrate, and the like.

Examples of the oxyacid esters include cetyl lactate, diisostearyl malate, hydrogenated castor oil monoisostearate, and the like.

Examples of the polyhydric alcohol fatty acid esters include glyceryl trioctanoate, glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate/eicosadioate, trimethylolpropane trioctanoate, trimethylolpropane triisostearate, neopentylglycol dioctanoate, neopentylglycol dicaprate, 2-butyl-2-ethyl-1,3-propanediol dioctanoate, propylene glycol dioleate, pentaerythrityl tetraoctanoate, pentaerythrityl hydrogenated rosin, ditrimethylolpropane triethyl hexanoate, ditrimethylolpropane (isostearate/sebacate), pentaerythrityl triethyl hexanoate, dipentaerythrityl (hydroxystearate/stearate/rosinate), diglyceryl diisostearate, polyglyceryl tetraisostearate, polyglyceryl-10 nonaisostearate, polyglyceryl-8 deca(erucate/isostearate/ricinoleate), Diglyceryl Sebacate/Isopalmitate, glycol distearate (ethylene glycol distearate), 3-methyl-1,5-pentanediol dineopentanoate, 2,4-diethyl-1,5-pentanediol dineopentanoate, and the like.

Examples of the silicones include dimethicone (dimethylpolysiloxane), highly polymerized dimethicone (highly polymerized dimethylpolysiloxane), cyclomethicone (cyclodimethylsiloxane, decamethylcyclopentasiloxane), phenyl trimethicone, diphenyl dimethicone, phenyl dimethicone, stearoxypropyl dimethylamine, (aminoethylaminopropyl methicone/dimethicone) copolymers, dimethiconol, dimethiconol crosspolymers, silicone resins, silicone rubber, amino-modified silicones such as aminopropyl dimethicone and amodimethicone, cation-modified silicones, polyether-modified silicones such as dimethicone copolyol, polyglycerol-modified silicones, sugar-modified silicones, carboxylic acid-modified silicones, phosphoric acid-modified silicones, sulfuric acid-modified silicones, alkyl-modified silicones, fatty acid-modified silicones, alkyl ether-modified silicones, amino acid-modified silicones, peptide-modified silicones, fluorine-modified silicones, cation-modified or polyether-modified silicones, amino-modified or polyether-modified silicones, alkyl-modified or polyether-modified silicones, polysiloxane/oxyalkylene copolymers, and the like.

Examples of the higher alcohols include cetanol, myristyl alcohol, oleyl alcohol, lauryl alcohol, cetostearyl alcohol, stearyl alcohol, arachidyl alcohol, behenyl alcohol, jojoba alcohol, chimyl alcohol, selachyl alcohol, batyl alcohol, hexyldecanol, isostearyl alcohol, 2-octyldodecanol, dimer diol, and the like.

Examples of the higher fatty acids include lauric acid, myristic acid, palmitic acid, stearic acid, isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisoheneicosanoic acid, long-chain branched fatty acid, dimer acid, hydrogenated dimer acid, and the like.

Examples of the fluorine-based oils include perfluorodecane, perfluorooctane, perfluoropolyether, and the like.

Examples of the polyhydric alcohol include ethanol, isopropanol, glycerin, propylene glycol, 1,3-butylene glycol, polyethylene glycol, and the like.

Furthermore, examples of the other bases include, but are not particularly limited to, bases used for adhesive preparations such as tape preparations such as plaster preparations or plasters (reservoir type, matrix type, etc.), poultices, patches or microneedles, ointments, external liquid preparations (liniments, lotions, etc.), spray preparations (external aerosols, pump spray preparations, etc.), creams, gels, eye drops, eye ointments, nasal drops, suppositories, rectal semisolids, enema agents, oral agents, injections, and the like.

Next, the present invention will be clarified by way of specific examples and comparative examples of the present invention. The present invention is not limited to the following examples.

(Example 1)

Preparation of core-shell structure;

One gram of donepezil hydrochloride (manufactured by Tokyo Chemical Industry Co., Ltd., octanol/water partition coefficient: 4.3, molecular weight: 416 g/mol) was dissolved in 40 g of pure water, and a solution obtained by dissolving 2 g of glyceryl caprylate (manufactured by Taiyo Kagaku Co., Ltd., product name "Sunsoft No. 700P-2-C", HLB value: 10.8, carbon number of saturated hydrocarbon group: 7) as a surfactant in 80 g of cyclohexane was added thereto, followed by stirring with a homogenizer (25,000 rpm, 2 min). Thereafter, the resultant was freeze-dried for 2 days to obtain a core-shell structure.

Preparation of formulation;

Into 30 parts by weight of the obtained core-shell structure, 70 parts by weight of an ointment base, plastibase (manufactured by Taisho Pharmaceutical Co., Ltd.), was formulated, and the mixture was mixed and dispersed to prepare a formulation (ointment).

(Example 2)

A formulation (ointment) was prepared in the same manner as in Example 1, except that 2 g of glyceryl caprylate (manufactured by ABITEC Corporation, product name "MCM-C8", HLB value: 10.1, carbon number of saturated hydrocarbon group: 7) was used as a surfactant, instead of 2 g of glyceryl caprylate (manufactured by Taiyo Kagaku Co., Ltd., product name "Sunsoft No. 700P-2-C", HLB value: 10.8, carbon number of saturated hydrocarbon group: 7).

(Example 3)

A formulation (ointment) was prepared in the same manner as in Example 1, except that 1 g of glyceryl caprylate (manufactured by ABITEC Corporation, product name "Capmul MCM-C8", HLB value: 10.1, carbon number of saturated hydrocarbon group: 7) and 1 g of diglyceride caprylate (manufactured by Taiyo Kagaku Co., Ltd., product name "Sunfat GDC-S", HLB value: 8.9, carbon number of saturated hydrocarbon group: 7) were used as surfactants, instead of 2 g of glyceryl caprylate (manufactured by Taiyo Kagaku Co., Ltd., product name "Sunsoft No. 700P-2-C", HLB value: 10.8, carbon number of saturated hydrocarbon group: 7).

(Example 4)

A formulation (ointment) was prepared in the same manner as in Example 1, except that 0.5 g of glyceryl caprylate (manufactured by ABITEC Corporation, product name "Capmul MCM-C8", HLB value: 10.1, carbon number of saturated hydrocarbon group: 7) and 1.5 g of diglyceride caprylate (manufactured by Taiyo Kagaku Co., Ltd., product name "Sunfat GDC-S", HLB value: 8.9, carbon number of saturated hydrocarbon group: 7) were used as surfactants, instead of 2 g of glyceryl caprylate (manufactured by Taiyo Kagaku Co., Ltd., product name "Sunsoft No. 700P-2-C", HLB value: 10.8, carbon number of saturated hydrocarbon group: 7).

(Example 5)

Preparation of core-shell structure;

One gram of octreotide acetate (manufactured by Shanghai Haoyuan Chemexpress Co., Ltd., octanol/water partition coefficient: 0.12, molecular weight: 1139.4 g/mol) was dissolved in 40 g of pure water, and a solution obtained by dissolving 2 g of glyceryl caprate (manufactured by ABITEC Corporation, product name "Capmul MCM-C10", HLB value: 8.9, carbon number of saturated hydrocarbon group: 9) as a surfactant in 80 g of cyclohexane was added thereto, followed by stirring with a homogenizer (25,000 rpm, 2 min). Thereafter, the resultant was freeze-dried for 2 days to obtain a core-shell structure.

Preparation of formulation;

Into 30 parts by weight of the obtained core-shell structure, 70 parts by weight of liquid paraffin (manufactured by Wako Pure Chemical Industries, Ltd.) was formulated, and the mixture was mixed and dispersed to prepare a formulation (lotion).

(Example 6)

Twenty parts by weight of acrylic pressure sensitive adhesive 1 (manufactured by CosMED Pharmaceutical Co. Ltd., product name "MAS683"), 30 parts by weight of acrylic pressure sensitive adhesive 2 (manufactured by CosMED Pharmaceutical Co. Ltd., product name "MAS811B"), and 10 parts by weight of a tackifier (manufactured by ARAKAWA CHEMICAL INDUSTRIES, LTD., product name "KE-311") were formulated into 40 parts by weight of the core-shell structure obtained in Example 2, and toluene was added thereto so that the concentration of the solid content was 35% by weight and then mixed until the mixture became uniform to prepare a pressure-sensitive adhesive layer solution.
Next, a release sheet subjected to a mold release treatment by application of silicone onto one surface of a release base material made of a polyethylene terephthalate film with a thickness of 38 μm was prepared. The pressure-sensitive adhesive layer solution was applied onto the surface subjected to the mold release treatment of this release sheet and dried at 90°C for 20 minutes to prepare a laminate having a pressure-sensitive adhesive layer with a thickness of 100 μm formed on the surface of the release sheet subjected to the mold release treatment. Then, a support made of a polyethylene terephthalate film with a thickness of 38 μm was prepared. One surface of the support and the pressure-sensitive adhesive layer of the laminate were superposed so as to face each other, and the pressure-sensitive adhesive layer of the laminate and the support were integrally laminated by transferring the pressure-sensitive adhesive layer onto the support to prepare a formulation (tape preparation).

(Comparative Example 1)

A formulation (ointment) was prepared in the same manner as in Example 1, except that 2 g of diglyceride caprylate (manufactured by Taiyo Kagaku Co., Ltd., product name "Sunfat GDC-S", HLB value: 8.9, carbon number of saturated hydrocarbon group: 7) was used as a surfactant, instead of 2 g of glyceryl caprylate (manufactured by Taiyo Kagaku Co., Ltd., product name "Sunsoft No. 700P-2-C", HLB value: 10.8, carbon number of saturated hydrocarbon group: 7) .

(Comparative Example 2)

A formulation (lotion) was prepared in the same manner as in Example 5, except that 2 g of diglyceride caprylate (manufactured by Accu Standard Inc., product name "Dicaprin", HLB value: 7.3, carbon number of saturated hydrocarbon group: 9) was used as a surfactant, instead of 2 g of glyceryl caprate (manufactured by ABITEC Corporation, product name "Capmul MCM-C10", HLB value: 8.9, carbon number of saturated hydrocarbon group: 9).

(Comparative Example 3)

A formulation (tape preparation) was prepared in the same manner as in Example 6, except that 2 g of diglyceride caprylate (manufactured by Taiyo Kagaku Co., Ltd., product name "Sunfat GDC-S", HLB value: 8.9, carbon number of saturated hydrocarbon group: 7) was used as a surfactant, instead of 2 g of glyceryl caprylate (manufactured by ABITEC Corporation, product name "Capmul MCM-C8", HLB value: 10.1, carbon number of saturated hydrocarbon group: 7).

(Reference Example 1)

An ointment base, plastibase (manufactured by Taisho Pharmaceutical Co., Ltd.), was used as it was.

(Reference Example 2)

Into 10 parts by weight of donepezil hydrochloride (manufactured by Tokyo Chemical Industry Co., Ltd., octanol/water partition coefficient: 4.3, molecular weight: 416 g/mol), 90 parts by weight of an ointment base, plastibase (manufactured by Taisho Pharmaceutical Co., Ltd.) was formulated, and the mixture was mixed and dispersed to prepare a formulation

(Proportion of compound having two or more fatty acid bonds)

For the surfactants used in Examples 1 to 6 and Comparative Examples 1 to 3, a proportion (% by weight) of the compound having two or more fatty acid bonds was determined using gel permeation chromatography (GPC). When a plurality of surfactants was contained, they were mixed at the mixing ratio of the surfactants used in Examples, and subjected to GPC measurement. An elution curve of the obtained GPC was separated into monoglyceride and diglyceride, and a proportion of the diglyceride (glycerin fatty acid ester that is a compound having two or more fatty acid bonds) and a proportion of the monoglyceride (glycerin fatty acid ester that is a compound having one fatty acid bond) were determined from the ratio between the monoglyceride and the diglyceride. Note that, the GPC measurement was performed using an APC system (manufactured by Waters Corporation) with tetrahydrofuran (THF) as a developing medium at a column temperature of 40°C and a flow rate of 1.0 ml/min. As a detector, "RI, PDA" was used, and as columns, one "ACQUITY APC XT125 4.6 × 150 mm" and two "ACQUITY APC XT45 4.6 × 150 mm" manufactured by Waters Corporation connected in series were used. As standard polystyrene, "PStQuick" manufactured by Tosoh Corporation was used.

(Evaluation)

The formulations obtained in Examples 1 to 6, Comparative Examples 1 to 3 and Reference Examples 1 to 2 were evaluated for hairless rat skin permeability and rabbit skin primary irritation by the following tests.

Hairless rat skin permeability test;

A hairless rat skin (manufactured by Japan SLC, Inc., extracted from HWY/Slc 8 weeks of age) was set in a drug skin permeation test cell (Fig. 2). The formulations obtained in Examples 1 to 6, Comparative Examples 1 to 3 and Reference Examples 1 to 2 were each applied to the upper part of this device in 0.2 g (1.33 cm$^2$) for the ointment and lotion, and in a size of 1.33 cm$^2$ for the tape preparation. In addition, a solution containing $5 \times 10^{-4}$ M of NaH$_2$PO$_4$, $2 \times 10^{-4}$ M of Na$_2$HPO$_4$, $1.5 \times 10^{-4}$ M of NaCl and 10 ppm of gentamicin sulfate (manufactured by Wako Pure Chemical Industries, Ltd., G1658) in distilled water was adjusted to pH 7.2 with NaOH to prepare a buffer, and the buffer was placed in a receptor layer at the lower part. In addition, the device was set in a thermostatic chamber kept at 32°C after the start of the test. At a predetermined time after the start of the test, 1 ml of the liquid in the thermostatic chamber was taken from the receptor layer at the lower part, and immediately afterwards, 1 ml of liquid having the same composition was replenished. Methanol was added to each of the collected receptor solution samples to extract an eluted lipid and the like, and the extract was centrifuged. After the centrifugation, the concentration of the active ingredient in the supernatant was quantitatively determined by high performance liquid chromatography (HPLC). On the basis of the amount of the active ingredient quantitatively determined, lag time and cumulative amount permeated through the skin over 24 hours were calculated.

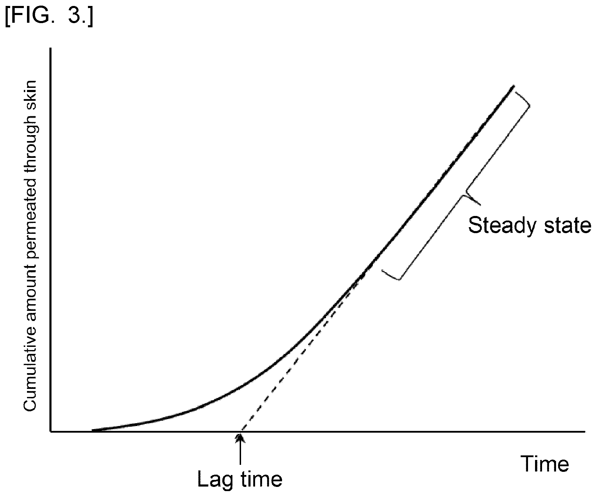

As shown in Fig. 3, in a graph having the cumulative amount permeated through the skin on the vertical axis and the time on the horizontal axis, the lag time is a time at which an extrapolated straight-line portion in a steady state crosses the horizontal axis.

Rabbit skin primary irritation test;

The dorsal skin of a rabbit was shaved with an electric clipper (shaved with an electric shaver as required). Healthy skin at two points on either side of the dorsal midline of the dorsal skin, that is, at four points in total, was used as administration sites. The formulations obtained in Examples 1 to 6, Comparative Examples 1 to 3 and Reference Examples 1 to 2 were taken with a spatula and spread evenly on pieces of lint each having a size of 2 cm $\times$ 2 cm, and the pieces were attached onto the administration sites. The lint pieces were fixed by covering with a non-woven adhesive bandage (manufactured by Nichiban Co., Ltd., MESHPORE, No. 50). Thereafter, the administration sites were entirely wrapped with gauze and then sealed by covering with an adhesive cloth elastic bandage (manufactured by Nichiban Co., Ltd., ELASTPORE, No. 100). The sealing was terminated 24 hours after the start of the administration, and the administered specimens were removed.

Skin reaction at 24 hours after the administration (30 minutes after the sealing was terminated and the administered specimens were removed) was observed by the naked eye. Thereafter, skin reaction at 48 hours and 72 hours after the administration (30 minutes after the sealing was terminated and the administered specimens were removed) was further observed by the naked eye in the same manner. Note that, the skin reaction was evaluated according to the criteria of Draize shown in Table 1 below.

[Table 1]

| Degree of skin reaction | Score |
| --- | --- |
| Erythema and eschar formation | |
| No erythema | 0 |
| Very slight erythema (barely perceptible) | 1 |
| Well-defined erythema | 2 |
| Moderate to severe erythema | 3 |
| Deep-red severe erythema with slight eschar formation (injuries in depth) | 4 |
| Edema formation | |
| No edema | 0 |
| Very slight edema (barely perceptible) | 1 |
| Well-defined edema (clearly distinguishable from surroundings) | 2 |
| Moderate edema (raised approximately 1 mm) | 3 |
| Severe edema (raised 1 mm or more and extending to periphery) | 4 |

Specifically, individual skin reaction scores (sum of erythema and eschar formation and edema formation) at the administration sites of each rabbit were calculated for each administered specimen at each observation time. Thereafter, the primary irritation index (P.I.I.) was calculated from the individual scores at 24 hours and 72 hours after the administration (the score at 48 hours after the administration was not added). Specifically, the following equations (1) and (2) were used for the calculation.

```
    Average score of each administration site = (Sum of
individual scores at 24 hours and 72 hours after
administration)/2 ... Equation (1)

    Primary irritation index (P.I.I.) = (Sum of average
score of each administration site)/(3 (rabbits)) ...
Equation (2)
```

From the primary irritation index (P.I.I.) obtained, the degree of irritation of each administered specimen was classified in accordance with the classification table of Table 2 below.

[Table 2]

| Primary irritation index (P.I.I.) | Safety classification |
| --- | --- |
| 0 | No irritation |
| 0 < P.I.I. $\leqq$ 2 | Slight irritation |
| 2 < P.I.I. $\leqq$ 5 | Moderate irritation |
| 5 < P.I.I. | Severe irritation |

The cumulative amount permeated through the skin over 24 hours, lag time, and skin irritation obtained by the above methods were evaluated according to the following evaluation criteria.

[Evaluation criteria]

Cumulative amount permeated through the skin over 24 hours;
AA ... 500 $\mu$g/cm$^2$ or more
A ... 300 $\mu$g/cm$^2$ or more and less than 500 $\mu$g/cm$^2$
C ... Less than 300 $\mu$g/cm$^2$

Lag time;
AA ... less than 4 hrs
A ... 4 hrs or more and less than 6 hrs
B ... 6 hrs or more and less than 8 hrs
C ... 8 hrs or more

Skin irritation (irritation);
AA ... Less than 1.8
A ... 1.8 or more and less than 2.8
C ... 2.8 or more

The results are shown in Table 3 below.

[Table 3]

| | Proportion of compound having two or more fatty acid bonds (% by weight) | Proportion of compound having one fatty acid bond (% by weight) | Cumulative amount permeated through skin over 24 hours ($\mu g/cm^2$) | | Lag time (hr) | | Irritation (P.I.I.) | |
|---|---|---|---|---|---|---|---|---|
| Example 1 | 6 | 94 | 1190 | AA | 6.4 | B | 0.8 | AA |
| Example 2 | 33 | 67 | 793 | AA | 1.4 | AA | 1.5 | AA |
| Example 3 | 58 | 42 | 598 | AA | 3.2 | AA | 2.5 | A |
| Example 4 | 71 | 29 | 445 | A | 5.9 | A | 2.7 | A |
| Example 5 | 30 | 70 | 1620 | AA | 4.3 | A | 2.1 | A |
| Example 6 | 33 | 67 | 503 | AA | 2.8 | AA | 1.6 | AA |
| Comparative Example 1 | 83 | 17 | 131 | C | 4.3 | A | 3.0 | C |
| Comparative Example 2 | 98 | 2 | 155 | C | 10.1 | C | 3.2 | C |
| Comparative Example 3 | 83 | 17 | 24 | C | 8.7 | C | 3.1 | C |
| Reference Example 1 | - | - | - | - | - | - | 0.0 | AA |
| Reference Example 2 | - | - | 9 | C | 18.2 | C | 0.5 | AA |

## EXPLANATION OF SYMBOLS

1: Core-shell structure
2: Core portion
3: Shell portion
11: Parafilm
12: Skin
13: Formulation
14: Receptor solution (pH = 7.2 phosphate buffer)
15: Stirrer

## Claims

1. A formulation comprising:

   a base; and
   a core-shell structure having a core portion containing an active ingredient and a shell portion containing a surfactant;
   the core portion being a solid,
   the surfactant containing a compound having two or more fatty acid bonds and a compound having one fatty acid bond, and
   a proportion of the compound having two or more fatty acid bonds in the surfactant being 5% by weight or more and 75% by weight or less.

2. The formulation according to claim 1, wherein the proportion of the compound having two or more fatty acid bonds in the surfactant is 10% by weight or more and 70% by weight or less.

3. The formulation according to claim 1 or 2, wherein the compound having two or more fatty acid bonds contains at least one of an ester of two or more fatty acids and a polyhydric alcohol and an ester of two or more fatty acids and a sugar or sugar derivative.

4. The formulation according to any one of claims 1 to 3, wherein the compound having two or more fatty acid bonds contains at least one of an ester of two or more fatty acids and glycerin and an ester of two or more fatty acids and sorbitan.

5. The formulation according to claim 4, wherein the compound having two or more fatty acid bonds contains an ester of two or more fatty acids and glycerin.

6. The formulation according to any one of claims 1 to 5, wherein the surfactant has a weighted average value of an HLB value of 4 or more and 14 or less.

7. The formulation according to any one of claims 1 to 6, wherein the surfactant has a saturated hydrocarbon group having 7 to 15 carbon atoms or an unsaturated hydrocarbon group having 7 to 17 carbon atoms.

8. The formulation according to any one of claims 1 to 7, wherein the mass ratio between the active ingredient and the surfactant (active ingredient : surfactant) is 1 : 0.1 to 1 : 10.

9. The formulation according to any one of claims 1 to 8, wherein the base is an oily base.

[FIG. 1.]

[FIG. 2.]

[FIG. 3.]

## INTERNATIONAL SEARCH REPORT

| International application No. |
| --- |
| PCT/JP2020/034123 |

### A. CLASSIFICATION OF SUBJECT MATTER
A61K 9/50(2006.01)i; A61K 47/14(2006.01)i; A61K 31/451(2006.01)i
FI: A61K47/14; A61K31/451; A61K9/50
According to International Patent Classification (IPC) or to both national classification and IPC

### B. FIELDS SEARCHED
Minimum documentation searched (classification system followed by classification symbols)
A61K9/50; A61K47/14; A61K31/451

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X | WO 2018/147333 A1 (SEKISUI CHEMICAL CO., LTD.) 16 August 2018 (2018-08-16) examples 1, 3, 6, 10, paragraphs [0007], [0128], examples 1, 26-27, table 7 | 1-9 |
| X | WO 2006/025583 A1 (ASPION CO., LTD.) 09 March 2006 (2006-03-09) claims 1, 5-6, production example 1, test example 1, fig. 1 | 1-9 |
| A | JP 2014-172840 A (LINTEC CORP.) 22 September 2014 (2014-09-22) claims, examples | 1-9 |
| A | JP 2010-260005 A (KANEKA CORP.) 18 November 2010 (2010-11-18) claims, examples | 1-9 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 12 October 2020 (12.10.2020) | 27 October 2020 (27.10.2020) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

| International application No. |
| --- |
| PCT/JP2020/034123 |

| Patent Documents referred in the Report | Publication Date | Patent Family | Publication Date |
| --- | --- | --- | --- |
| WO 2018/147333 A1 | 16 Aug. 2018 | US 2019/0117777 A1 claims 1, 4, 6, 10, paragraphs [0007], [0139], examples 1, 26-27, table 7 EP 3453404 A1 | |
| WO 2006/025583 A1 | 09 Mar. 2006 | US 2009/0238846 A1 claims 1, 5-6, production example 1, test example 1, fig. 1 EP 1785131 A1 | |
| JP 2014-172840 A | 22 Sep. 2014 | (Family: none) | |
| JP 2010-260005 A | 18 Nov. 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018147333 A